# EUROPÄISCHE PATENTANMELDUNG

(11) **EP 1 574 983 A2**
(43) Veröffentlichungstag der Anmeldung: **14.09.2005**
(21) Anmeldenummer: 05004904.8
(22) Anmeldetag: 07.03.2005
(51) Int. Cl.: G06F 19/00

(54) **Verfahren und System zum Erfassen der Ergebnisse eines psychologischen Tests**

(30) Priorität: 08.03.2004 DE 102004011124
(71) Anmelder: Dries, Christian, Dr., 50679 Köln (DE)
(72) Erfinder: Dries, Christian, Dr., 50679 Köln (DE)
(74) Vertreter: Patentanwälte Freischem

(57) **Zusammenfassung**

Die Erfindung betrifft ein Verfahren und ein System zur Erfassung der Ergebnisse eines psychologischen Tests. Insbesondere betrifft sie ein Verfahren und ein System zur Erfassung der Ergebnisse eines Tests zur Personalbeurteilung durch mindestens einen Beobachter.

Aufgabe der Erfindung ist es, ein Verfahren und ein System zur Erfassung der Ergebnisse eines psychologischen Tests zu schaffen, welches den Ablauf des Tests und die Aussagekraft der Testergebnisse fördert.

Diese Aufgabe wird dadurch gelöst, daß auf einer Anzeigevorrichtung eines Computers Eingabefelder erzeugt werden, der Beobachter die Ergebnisse in die Eingabefelder einträgt, die Ergebnisse in einem Datenspeicher abgespeichert werden und der Computer zusätzliche Funktionen der Ergebniserfassung und/oder Testdurchführung automatisch durchführt.

Die Erfindung betrifft ferner Computerprogrammprodukt und ein Computersystem zur Durchführung des Verfahrens.

## Beschreibung

### Technisches Gebiet

Die Erfindung betrifft ein Verfahren und ein System zur Erfassung der Ergebnisse eines psychologischen Tests. Insbesondere betrifft sie ein Verfahren und ein System zur Erfassung der Ergebnisse eines Tests zur Personalbeurteilung durch mindestens einen Beobachter.

### Stand der Technik

In verschiedensten Bereichen werden heutzutage Tests zur Beurteilung der psychischen und intellektuellen Fähigkeiten von Personen durchgeführt. Eine besondere wirtschaftliche Bedeutung kommt derartigen psychologischen Tests bei der Beurteilung verschiedener Bewerbungen für eine Stelle in einem Unternehmen, das heißt bei der Personalbeurteilung zu. Die Beurteilung von Bewerbern für Posten in großen Unternehmen und insbesondere für Führungspositionen wird heute zunehmend in sogenannten Beurteilungszentren, international auch "Assessment-Center" genannt, durchgeführt. Ein Assessment-Center dient der Beurteilung und Einschätzung der Fähigkeiten und Kompetenzen einzelner Bewerber auf der Grundlage anerkannter und bewährter psychologischer Tests. Assessment-Center gliedern sich in der Regel in mehrere Teilbereiche auf, in denen unterschiedliche Tests durchgeführt werden. Derartige Tests sind beispielsweise Rollenspiele, Planspiele, Gruppendiskussionen, Einzelvorträge, Fallstudien und psychologische Testverfahren. Dabei werden die Kandidaten in Situationen (Übungen) beobachtet, die die Anforderungen der künftigen Aufgabe so gut wie möglich abbilden sollen. Mehrere Beobachter halten anhand von Beobachtungsbögen das Verhalten der Teilnehmer in jeder Situation fest. Verschiedene Eigenschaften und Fähigkeiten jedes Teilnehmers werden von den Beobachtern beurteilt. Die verschiedenen Anforderungen werden auf einer Skala bewertet, was den Vergleich der verschiedenen Teilnehmer untereinander erlaubt. Auch können durch die Skalierung Leistungsunterschiede einzelner Kandidaten in verschiedenen Übungen beobachtet werden.

Die Beobachter bedienen sich dabei in der Regel vorbereiteter Beobachtungsbögen, in denen Beurteilungen und Beobachtungen handschriftlich eingetragen werden. Die erfaßten Daten werden im Rahmen einer sogenannten Beobachterkonferenz zusammengetragen und zu einer endgültigen Bewertung des Bewerbers zusammengefaßt.

Während sich für die Erfassung der intellektuellen Fähigkeiten sowie des Bildungsstandes der Bewerber computergestützte Tests, zum Beispiel zur Erfassung des mathematischen Wissens, des naturwissenschaftlichen Wissens, der Sprachkenntnisse, bewährt haben, haben sich in der Praxis für die Beurteilung der charakterlichen Eigenschaften und psychologischen Leistungsfähigkeit der Bewerber keine automatisierten Verfahren als so leistungsfähig und aussagekräftig erwiesen, wie die Beurteilung des Bewerberverhaltens durch geschulte Beobachter in Übungssituationen. Auch sind automatische Verfahren zur Standardisierung und zum Vergleich der Daten über einzelne Bewerber bekannt, ohne daß hierbei der Vorgang der Informationsgewinnung, das heißt der Erfassung der Testergebnisse durch mindestens einen Beobachter, angesprochen ist.

So beschreibt beispielsweise das Dokument US 2002/0055870 ein Verfahren und eine Vorrichtung für den Vergleich von Personenbewertungen mit Bewertungsanforderungen. Der Vorgang der Datengewinnung, insbesondere der Personalbeurteilung durch psychologisch geschulte Beobachter, ist hier kaum angesprochen. Es wird weitgehend von den Daten ausgegangen, die der Bewerber über seine Person selbst angibt.

Ein vollautomatisches Befragungssystem und -verfahren geht aus der Druckschrift WO 02/21303 hervor. Dieses Verfahren mag die automatische Abarbeitung der Ergebnisse von über einen Computer geführten Interviews und somit die schnelle Auswertung einer Vielzahl von Befragungs-Ergebnissen ermöglichen. Dagegen wird nicht die Aussagekraft und Zuverlässigkeit individueller Befragungen und Tests durch psychologisch geschulte Beobachter erreicht. Ähnliches gilt für die Vorrichtung der WO 02/13095. Hier sind der automatischen Antworterfassung automatische Vorhersagemittel zugeordnet, welche die Eignung individueller Bewerber für eine Anstellung ermitteln sollen.

Die Druckschrift WO 03/009187 beschreibt ein internetgestütztes Personalbeurteilungssystem. Hier werden über das Internet Anforderungsprofile der Arbeitgeber und Prüfergebnisse der Personalberater zusammengeführt. Auch die Bewerber haben Zugriff auf dieses System. In diesem Dokument ist ausführlich die Erstellung von Anforderungsprofilen sowie der Vergleich der Anforderungsprofile mit Bewertungsprofilen beschrieben, um als Ergebnis eine Übereinstimmung festzustellen. Die Frage der Erfassung der Leistungen und Bewertungsprofile der Bewerber ist weniger gründlich behandelt. Es wird vorgeschlagen, z.B. das technisch-wissenschaftliche Wissen durch einen Online-Test zu erfassen. Auch ist angesprochen, daß einige Kompetenzen des Bewerbers in einem persönlichen Gespräch, hier Interview genannt, erfaßt werden müssen. Die Resultate dieses Interviews werden nach Beendigung des Interviews in ein auf einem Computer gespeicherten Profil des Bewerbers eingetragen. Das Dokument behandelt nicht die Frage, wie die Bewertungsprofile während eines Interviews durch einen psychologisch geschulten Personalberater erfaßt werden.

### Offenbarung der Erfindung

Aufgabe der Erfindung ist es, ein Verfahren und ein System zur Erfassung der Ergebnisse eines psychologischen Tests zu schaffen, welches den Ablauf des Tests und die Aussagekraft der Testergebnisse fördert.

Diese Aufgabe wird durch ein Verfahren mit der Gesamtheit der Merkmale des Patentanspruchs 1 gelöst.

In Abkehr von der üblichen Praxis, die vom Beobachter notierten Testergebnisse handschriftlich festzuhalten, wird zur Erfassung der Ergebnisse ein Computer verwendet, auf dessen Anzeige Eingabefelder erzeugt werden, in welche die Ergebnisse eingetragen werden können, um in einem Datenspeicher abgespeichert zu werden. Hierdurch wird bereits die Verfügbarkeit der Ergebnisse erheblich gesteigert. So kann ein Aufseher oder Supervisor bereits während des Testes durch Einsicht in die abgespeicherten Ergebnisse den Eindruck des oder der Beobachter kennenlernen. Zusätzlich führt der Computer Funktionen der Ergebniserfassung und/oder Testdurchführung automatisch durch.

Natürlich betrifft die Erfindung auch die Verwendung eines Computers zur Erfassung der Ergebnisse eines psychologischen Tests, insbesondere eines Tests zur Personalbeurteilung, durch mindestens einen Beobachter und zur automatischen Durchführung mindestens einer zusätzlichen Funktionen der Ergebniserfassung und/oder Testdurchführung. Bei dieser Verwendung werden vorzugsweise die Merkmale des vorgenannten und auch des nachfolgend beschriebenen Verfahrens verwirklicht.

Bei einer ersten praktischen Ausführungsform erfaßt der Computer die laufende Zeit des Tests, d.h. die seit dem Beginn des Tests abgelaufene Zeit. Durch diese Zeiterfassung kann mittels des Computers zusammen mit einem Eintrag in ein Eingabefeld die im Zeitpunkt des Eintrags abgelaufene Zeit des Tests abgespeichert werden. Die einem Eintrag zugeordnete Zeit des Tests hat sowohl eine hohe Aussagekraft über den Bewerber als auch über den Beobachter. Eigenschaften, die der Beobachter in einem frühen Zeitpunkt einträgt, scheinen für den Beobachter offensichtlich. So mag eine frühzeitig erkannte Selbstsicherheit und ein frühzeitig erkanntes kompetentes Auftreten eines Bewerbers in bezug auf bestimmte Anstellungsvoraussetzungen einen anderen Stellenwert haben, als die gleiche Selbstsicherheit und Kompetenz, die erst im Laufe eines längeren Gesprächs festgestellt wird. Der Wert einer Eintragung kann also mit dem automatisch erfaßten Zeitwert gewichtet werden.

Eintragungen zur Unzeit können allerdings auch Aufschluß über eine voreilige oder fehlerhafte Beurteilung geben. Bestimmte Qualitäten und Eigenschaften eines Probanden lassen sich zuverlässig erst am Ende eines bestimmten Testabschnitts bestimmen. Werden Eintragungen zu diesen Merkmalen frühzeitig durchgeführt, kann hieraus abgeleitet werden, daß der Beobachter seine Beurteilung vorschnell abgegeben hat. Für Ergebnisse, die erst nach einer bestimmten Testzeit erfaßt werden dürfen, können bei einer Ausführungsform der Erfindung durch den Computer die betroffenen Eingabefelder erst nach einer vorbestimmten Zeit für die Aufnahme eines Eintrags freigegeben werden.

Das Ergebnis zu einem bestimmten Kriterium, insbesondere einer Qualifikation oder Eigenschaft einer Testperson, kann erfindungsgemäß in Form eines skalierten Wertes erfaßt werden. Ein skalierter Wert ist in der Regel ein Wert, der zwischen einem Minimalwert und einem Maximalwert liegt. Bei der Beurteilung bestimmter Eigenschaften von Testpersonen kann es beispielsweise sinnvoll, Bewertungen in sechs unterschiedlichen Abstufungen von weit überdurchschnittlich (= 6) bis weit unterdurchschnittlich (=1) zu erfassen. Bei einer praktischen Ausführungsform können diese in Form eines skalierten Wertes erfassende Ergebnisse zu einem bestimmten Kriterium mehrmals zu unterschiedlichen Zeiten erfaßt werden. Die Summe aller dem genannten Kriterium zugeordneten Ergebnisse kann durch die Anzahl der Eingaben geteilt werden, so daß das arithmetische Mittel aller Eingaben gebildet wird. Auf diese Weise wird zu einem bestimmten Kriterium nicht nur das Ergebnis erfaßt, welches gegen Ende des Testes durch den Beobachter festgestellt wird. Es werden zu diesem Kriterium auch Ergebnisse am Anfang des Testes und während des Testablaufs erfaßt. Diese über den Testablauf verteilte Ergebniserfassung wirkt einem Effektentgegen, der bei der Erfassung nur des Endergebnisses auftritt. Wird nur das Endergebnis erfaßt, überwiegt in der Regel der letzte Eindruck des Beobachters; Durch die Erfassung des Ergebnisses während des Testverlaufs werden auch die Eindrücke des Beobachters zu Beginn des Testes und in der Mitte des Testes hinreichend berücksichtigt. Zudem kann die Anzahl der Einträge zu einem bestimmten Kriterium Rückschlüsse darauf zulassen, wie stark der Eindruck eines Beobachters in bezug auf dieses bestimmte Kriterium ist.

In der Praxis können die zu jedem bestimmten Kriterium eingegebenen Ergebnisse zusammen mit dem Zeitpunkt ihrer Eingabe abgespeichert werden. Dies ermöglicht einerseits die Zuordnung bestimmter Beurteilung zu bestimmten Momenten des Tests. Zusätzlich wird eine Beurteilung des Beobachterverhaltens ermöglicht. Zum einen können die Einträge und die Zeitpunkte der Einträge verschiedener Beobachter verglichen werden. Insbesondere Einträgen, die nahezu zeitgleich von mehreren Beobachtern gemacht werden, kommt eine besondere Aussagekraft zu. Beobachter; deren Beobachtungen von denen anderer stark abweichen, können aufgrund dieser Informationen geschult werden. Dies ist insbesondere dann möglich, wenn die computerisierte Ergebniserfassung mit einer Videoaufnahme des Tests gekoppelt und synchronisiert wird, so daß die Beobachter die Zeitpunkte ihrer Eintragungen konkreten Ereignissen im Testablauf, die in der Videoaufnahme festgehalten sind, zuordnen können.

Ferner können Protokolle über Änderungen der Einträge durch den Beobachter ebenfalls eine Aussagekraft über den Bewerber haben, insbesondere, wenn in den Protokollen den jeweiligen Änderungen konkrete Zeitwerte zugeordnet sind. Vor allem, wenn ein Test von mehreren Beobachtern beurteilt wird, läßt sich durch Vergleich der verschiedenen automatischen Testprotokolle mit gekoppelter Zeitwerterfassung ermitteln, ob die Änderung einer Eigenschaft auf eine subjektive Fehlbeurteilung eines Beobachters oder aber auf eine Änderung des Verhaltens des Bewerbers oder des durch diesen erzeugten Eindrucks zurückzuführen ist. Letzteres muß bei mehreren Beobachtern zu einem etwa zeitgleichen Ändern des Eintrags in bezug auf bestimmte Eigenschaften führen.

Vorzugsweise werden die Testergebnisse von mehreren Beobachtern erfaßt, wobei deren Computer untereinander vemetzt sind. Darüber hinaus sind die Computer vorzugsweise mit einem Server vemetzt. Der Server dient der zentralen Datenabspeicherung. Wie oben erwähnt, kann ein Supervisor während der Durchführung des Tests Einblick in diese Daten auf dem Server nehmen und somit frühzeitig die Beurteilungen der Beobachter kennenlernen. Die Testergebnisse werden dabei vorzugsweise in eine gemeinsame Datenbank geschrieben.

Aus der gemeinsamen Datenbank kann in der Praxis automatisch eine Ergebnisdatei erstellt werden. Diese Ergebnisdatei kann entweder dem Supervisor während der Testdurchführung angezeigt werden oder am Ende des Testes für die Beobachterbe-sprechung oder zur Präsentation der Testergebnisse verwendet werden.

Eine weitere zusätzliche Funktion, die der Computer im Falle der Vernetzung automatisch zur Förderung der Testdurchführung übernehmen kann, ist die Kommunikations-Funktion. In der Praxis versendet hierbei mindestens ein Computer eine Nachricht über einen Datennetzwerk, die von mindestens einem anderen Computer empfangen und wiedergegeben wird. Durch diese Kommunikationsform können die Beobachter untereinander kommunizieren, ohne daß der Testablauf durch diese Kommunikation gestört wird. Beispielsweise können kurze Texte in ein Textfeld eingegeben werden und auf einem Anzeigefeld auf dem Bildschirm des Nachrichtenempfängers wiedergegeben werden. Der Computer, der die Nachrichten empfängt und anzeigt, muß nicht unbedingt einem Beobachter zugeordnet sein. Er kann auch derjenigen Person zugeordnet sein, die ein Gespräch führt oder einen Test durchführt, ohne als Beobachter aufzutreten. Insbesondere in diesem Fall können entweder die Beobachter oder ein Supervisor, der ebenfalls über einen an das Datennetz angeschlossenen Computer verfügt, Informationen und Anweisungen an die Person übermitteln, welche den Test durchführt oder das Gespräch führt. Dies geschieht, ohne daß der Bewerber die Kommunikation bemerken kann und sich hierdurch gestört fühlt. Die Nachricht muß nicht als Textnachricht übermittelt werden, sondern kann auch beliebige andere Formen haben und beispielsweise eine gesprochene Nachricht sein. In diesem Fall kann an den Computer eines Nachrichtensenders ein Mikrofon angeschlossen sein, an den Computer des Nachrichtenempfängers kann ein Kopfhörer angeschlossen sein, über den die über das Datennetzwerk übertragene Nachricht wiedergegeben wird, ohne daß der Bewerber dies hört.

In der Praxis werden die Eingaben in die Computer vorzugsweise über einen berührungsempfindlichen Bildschirm, einen sogenannten Touch-Screen, eingegeben. Insbesondere eignet sich als Computer für den Beobachter ein sogenannter Tablet PC. Es handelt sich hierbei um tragbare Computer, welche die üblichen Hardware-Komponenten herkömmlicher Notebooks aufweisen (Prozessor, Arbeitsspeicher, Festplattenlaufwerke, Datenschnittstelle, Bildschirm), bei dem der Bildschirm ein berührungsempfindlicher Touch-Screen ist, über den Dateneingaben gemacht werden können. Tablet PCs sind sowohl als sogenannte Convertibles bekannt, welche sich von einem herkömmlichen Notebook durch Drehen des Bildschirmes in einen Tablet PC mit oben liegender berührungsempfindlicher Bildschirmoberfläche verwandeln lassen. Unterhalb des Abschnittes mit dem Bildschirm liegt dann der Gehäuseabschnitt mit der Tastatur. Ein reiner Tablet PC verfügt über keine Tastatur und lediglich über das Gehäuse, in dem der Bildschirm sowie die anderen Hardware-Komponenten des Computers untergebracht sind.

Auf den berührungsempfindlichen Bildschirm können die Eingaben auf unterschiedliche Weise erfolgen. Für bestimmte Eigenschaften können nur eine begrenzte Anzahl von Werten (z.B. fünf unterschiedliche Werte) zulässig sein. In diesem Fall kann ein Eingabefenster unter Auflistung der fünf Werte auf dem Bildschirm des Computers dargestellt werden, wobei der gewählte Wert durch den Beobachter markiert und eingegeben wird. Eine schriftliche Eingabe kann über eine Tastatur erfolgen. Da in der Regel das Schreiben auf einer herkömmlichen Tastatur laut ist und den Test stören kann, kann auf dem berührungsempfindlichen Bildschirm eine Tastatur abgebildet sein, wobei die einzelnen Buchstaben durch Antippen der Abbildung der Buchstaben aktiviert werden.

Bei einer praktischen Ausführungsform erfolgen die Eingaben über einen berührungsempfindlichen Bildschirm handschriftlich: Moderne Tablet PCs sind mit leistungsfähigen Schrifterkennungsprogrammen ausgestattet. Bei einer reproduzierbaren Handschrift kann das Schrifterkennungsprogramm die handschriftliche Eingabe in einen alphanumerischen Text umwandeln und als Textdatensatz abspeichem. Eine Abbildung der handschriftlichen Eingabe kann zusätzlich oder alternativ als Bilddatensatz abgespeichert werden. Die kombinierte Abspeicherung der beiden unterschiedlichen Datensätze erlaubt die schnelle automatische Weiterverarbeitung der Textdatei sowie die Überprüfung der korrekten Schrifterkennung unter Zuhilfenahme des Bilddatensatzes. Ein weiterer Vorteil der Erfindung ist, daß als Textdatei gespeicherte Notizen sofort in ein Gutachten eingebunden werden können, welches auf der Grundlage der Testergebnisse erstellt wird. Bei einer weitere Ausführungsform kann ein Spracherkennungsprogramm auf dem Computer ausgeführt werden, um gesprochene Beobachtungen oder Bewertungen des Beobachters zu erkennen.

In der Praxis kann der Computer eines Beobachters mehrere Eingabemasken für mehrere Personen erzeugen, die während eines Testes gleichzeitig beobachtet werden. Jede der Eingabemasken kann von dem Beobachter durch Eingabe mit einem Eingabemittel, vorzugsweise durch Berühren des Touch-Screens an einer bestimmten Stelle mit einem Eingabestift, aktiviert werden.

Jede der Eingabemasken zeigt vorzugsweise den Namen einer bestimmten Person, der sie zugeordnet ist, in einem Identifizierungsfeld an. Durch Berühren dieses Identifizierungsfeldes kann die entsprechende Eingabemaske aktiviert werden. Zusätzlich oder alternativ ist es möglich, daß jede der Eingabemasken ein Bild der Person anzeigt, der sie zugeordnet ist. Durch die Anzeige des Bildes der Person kann vermieden werden, daß ein Beobachter Ergebnisse zu einer Person in eine falsche Eingabemaske einträgt.

Der Computer kann automatisch für jede Eingabemaske die Gesamtzeit erfassen, in der diese Eingabemaske durch den Beobachter während eines Tests aktiviert ist. Hierdurch kann festgestellt werden, wie intensiv sich ein Beobachter mit einer bestimmten Person beschäftigt hat. Der erfaßte Betrag der Gesamtzeit, in der die Eingabemaske einer bestimmten Person durch den Beobachter aktiviert war, kann dazu verwendet werden, die Eingaben dieses Beobachters zu dieser bestimmten Person zu gewichten.

In der Praxis kann außerdem mindestens eine Videoaufnahme von der Durchführung des Tests aufgezeichnet werden. Hierdurch ist es möglich, Beobachter in die Testdurchfühnmg einzubinden, die von dem Ort des Tests entfernt sind. Die Videoaufnahme des Tests kann über das Internet oder über andere Übertragungswege (Fernseh-Satelliten etc.) zu einem Empfänger an beliebigen Orten der Welt übertragen werden, der sich die Aufnahme auf einem Bildschirm ansieht. Dieser Beobachter kann wie die Beobachter vor Ort die Ergebnisse in Eingabefelder seines Computers eintragen. Durch die Zusammenführung der eingetragenen Ergebnisse über das Internet in eine zentrale Ergebnisdatei können die entfernt erstellten Ergebnisse genau so berücksichtigt werden wie die vor Ort erstellten Ergebnisse. Alle Ergebnisse liegen unmittelbar nach Beendigung eines Tests vor.

Alternativ kann eine Videoaufnahme zeitversetzt abgespielt werden und ein Beobachter die Ergebnisse somit zeitversetzt erzeugen. In diesem Fall liegen die Ergebnisse erst vor, wenn der letzte Beobachter die Ergebnisse bei der Betrachtung der Videoaufnahme erzeugt hat.

Einen weiteren Vorteil bietet die Videoaufnahme des Tests aber auch bei der Testauswertung, der Bewertung der Beobachter und der Präsentation der Testergebnisse. Bei einer Testauswertung in einer Beobachterkonferenz kann eine Gesamtbeurteilung nicht nur auf Grund der durch die Computer erfaßten Zeitpunkte der Einträge und die eingetragenen Ergebnisse erstellt werden. Diese Zeitpunkte und die Ergebnisse können auch konkreten Situationen während des Testablaufs zugeordnet werden. Dies läßt gleichzeitig eine Beurteilung der Leistung der Beobachter zu, so daß das erfindungsgemäße Verfahren auch vorteilhaft für die Beurteilung der Beobachtung im Rahmen einer Ausbildung oder Weiterbildung verwendet werden kann.

Schließlich erlaubt die Videoaufnahme bei der Präsentation der Testergebnisse vor einem Personalmanager, der bei dem Test nicht persönlich anwesend war, die Testergebnisse durch Präsentation des tatsächlichen Testverlaufes mittels der Videoaufnahme zu verdeutlichen.

Darüber hinaus betrifft die Erfindung ein Computerprogrammprodukt, welches zur Erfassung der Ergebnisse eines psychologischen Tests direkt in den Arbeitsspeicher eines Computers ladbar ist und Programmcodeabschnitte aufweist, welche beim Ablauf des Programms die Schritte des zuvor beschriebenen Verfahrens ausführen. Das Computerprogrammprodukt erzeugt einerseits auf der Anzeige des Computers die Eingabefelder und nimmt die Eintragungen des Beobachters auf und speichert sie in dem Datenspeicher ab. Zusätzlich enthält das Computerprogrammprodukt die erforderlichen Programmcodeabschnitte zur Durchführung der automatischen Funktionen der Ergebniserfassung und/oder der Testdurchführung.

Ferner umfaßt die Erfindung ein Computersystem zur Erfassung der Ergebnisse eines psychologischen Tests, insbesondere eines Tests zur Personalbeurteilung, durch mindestens einen Beobachter, welches für jeden Beobachter einen Computer umfaßt, auf dem ein Computerprogrammprodukt der genannten Art abläuft. In der Praxis kann der genannte Computer zur automatischen Zeiterfassung einen Timer, auch Zeitgeber genannt, umfassen. Wie erwähnt, weist der Computer vorzugsweise einen berührungsempfindlichen Bildschirm auf. Schließlich sollte der Computer ein Modul zur drahtlosen Datenübertragung aufweisen. Die Vernetzung der Computer verschiedener Beobachter untereinander sowie gegebenenfalls mit einem Server können in der Praxis über ein sogenanntes drahtloses Datennetz (WLAN = wireless local area network) erfolgen. Ein derartiges drahtloses Datennetz ermöglicht den Datenaustausch zwischen einem drahtlosen Zugangspunkt (wirless access point) und einem Computer über 30 Meter und mehr. Die einzelnen Beobachter sind somit durch den ihnen zugeordneten, vernetzten Computer in ihrer Beweglichkeit nicht eingeschränkt.

Die Architektur des Netzwerks kann beliebiger Art sein. In der Praxis hat sich die Verwendung eines lokalen Servers für das Netzwerk bewährt, der mit einem drahtlosen Zugangspunkt (wireless access point) verbunden ist. Beim Betrieb größerer Assessment-Center, bei denen unterschiedliche Tests in verschiedenen Räumen durchgeführt werden, kann der lokale Server auch über eine entsprechende Vernetzung mit mehreren drahtlosen Zugangspunkten verbunden sein, so daß eine zuverlässige Datenübertragung in allen Räumen des Assessment-Centers gewährleistet ist.

In der Praxis kann der lokale Server mit einem zentralen Server über ein Datennetzwerk, insbesondere das Internet verbunden sein. Diese Netzwerksarchitektur hat mehrere Vorteile. Professionelle Personalberater großer Unternehmen oder eigenständige Unternehmen, die auf die Personalberatung spezialisiert sind, können auf ihrem eigenen Server Informationen und Datensätze abspeichern, die für die Durchführung verschiedener psychologischer Tests erforderlich sind. Durch die Tatsache, daß ein mobiler Server immer über ein Datennetzwerk Zugriff auf den zentralen Server hat, ist gewährleistet, daß alle Assessment-Center, die von Mitarbeitern dieses Unternehmens betrieben werden, auf den gleichen aktuellen Datenbestand zugreifen. Ferner können die Testergebnisse nahezu in Echtzeit von dem mobilen Server auf den zentralen Server übermittelt werden. Hierdurch wird ein Datenverlust, beispielsweise durch eine Beschädigung des mobilen Servers, verhindert, insbesondere, wenn der zentrale Server über zuverlässige Datensicherungsmechanismen, beispielsweise einer Spiegelung seiner Festplatte, verfügt.

Die Erfindung führt zu einer beachtlichen Verbesserung der Zuverlässigkeit der Beurteilungserfassung durch menschliche Beobachter: Oben wurde bereits beschrieben, wie die Protokollierung des Verhaltens des Beobachters wesentliche Rückschlüsse auf die Qualität der Bewertung dieses Beobachters zuläßt. Darüber hinaus vermeidet die Verwendung von Computern die Übertragung der Eintragungen der jeweiligen Beobachter in Datensätze, welche auf Computern verarbeitet und gespeichert werden können. Hierdurch werden Übertragungsfehler vermieden. Außerdem entfallen die Kosten für die Arbeitsleistung der Datenübertragung. Schließlich stehen die Daten aller Beobachter unmittelbar nach Abschluß des Testes in einer Datenbank zur Verfügung. Dies ist insbesondere für eine häufig im Anschluß an einen Test durchgeführte Beobachterbesprechung von entscheidender Bedeutung. Neben den tatsächlichen Eingaben der Beobachter kann sofort nach Testbeendigung eine Datenanalyse stattfinden. Beispielsweise können die Übereinstimmungen der Beurteilungen verschiedener-Beobachter ermittelt werden. Sehr hohe oder sehr geringe Übereinstimmungen können automatisch in einem Datensatz oder bei einer Datenanzeige markiert werden, so daß sie bei einer Beobachterbesprechung auffallen und weiter erörtert werden können.

Ferner läßt sich die aktuell übliche Einschätzungsmethodik verbessern. Bisher werden die Beurteilungen durch die Beobachter jeweils einmal gegen Ende des Tests abgegeben. Die computerisierte Beurteilungserfassung ermöglicht mehrere Einträge zu unterschiedlichen Zeitpunkten in bezug auf ein Beurteilungskriterium, so daß ggf. unterschiedliche Eindrücke zu den verschiedenen Zeitpunkten protokolliert werden können und in die Gesamtbeurteilung einfließen. Dies wirkt dem bisher bekannten Effekt der Verfälschung des Ergebnisses durch den letzten Eindruck entgegen. Macht ein Bewerber am Ende des Tests einen sehr guten Eindruck, besteht die Gefahr, daß die Beurteilung zu gut ausfällt. Umgekehrt besteht die Gefahr eines zu schlechten Urteils im Falle eines schlechten Endeindrucks. Durch die Erfassung der Beurteilung einzelner Kriterien nicht nur einmal am Testende, sondern mehrmals während des Tests, kann dieser Ergebnisverfälschung entgegengewirkt werden. Bei einer praktischen Ausführungsform wird aus den über die gesamte Testzeit für ein bestimmtes Kriterium eingetragenen Ergebnissen ein Mittelwert gebildet.

Schließlich ist es möglich, die durch das erfindungsgemäße Verfahren erfaßten Daten in Personaldatenbanken, auch Human-Resource-Datenbanken genannt, zu übertragen, welche üblicherweise von den Personalabteilungen größerer Unternehmen geführt werden.

### Kurze Beschreibung der Zeichnungen

Praktische Ausführungsformen des erfindungsgemäßen Systems zur Durchführung des erfindungsgemäßen Verfahrens werden nachfolgend unter Bezugnahme auf die beigefügten Zeichnungen beschrieben. Die Zeichnungen zeigen in
- Figur 1: eine schematische Darstellung eines Computer-Systems zur Durchführung des erfindungsgemäßen Verfahrens,
- Figur 2: eine schematische Darstellung der Netzwerk-Konfigurierung des erfindungsgemäßen Systems in den Räumen eines Assessment-Centers,
- Figur 3: eine Darstellung der verschiedenen Programmmodule, die zur Durchführung der Erfindung zusammenwirken;
- Figur 4: ein erstes Beispiel für eine Eingabemaske zur Erfassung der Testergebnisse und
- Figur 5: ein zweites Beispiel für eine Eingabemaske zur Erfassung der Testergebnisse.

### Ausführungsformen der Erfindung

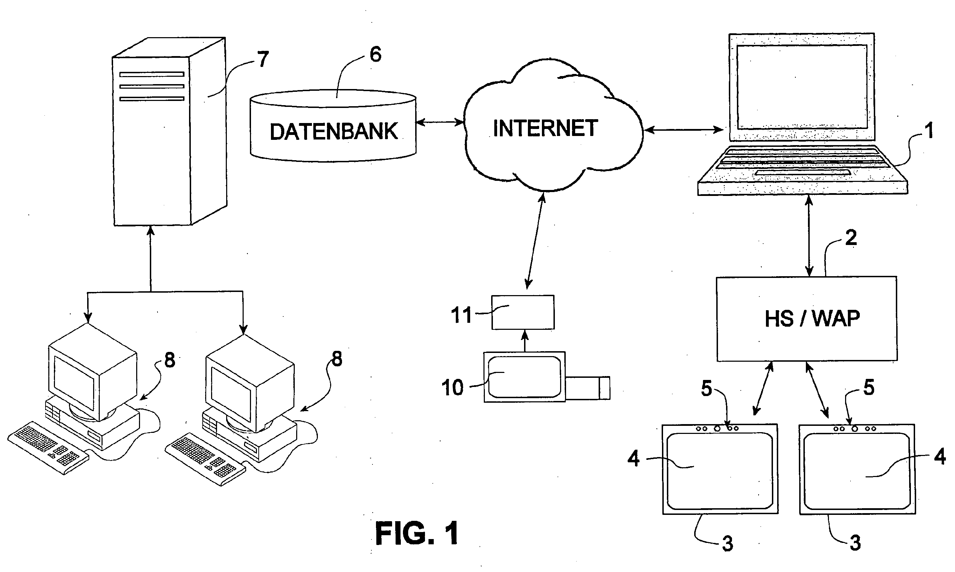

Die Figur 1 zeigt schematisch den Aufbau eines Computersystems, auf dem das erfindungsgemäße Verfahren zur digitalen Erfassung der Personalbeurteilung durchgeführt werden kann. Das Netzwerk zur Durchführung der digitalen Ergebniserfassung selbst ist im rechten Abschnitt der Figur 1 dargestellt. Das Netzwerk besteht aus einem mobilen Server 1, der im vorliegenden Fall als Notebook-Computer dargestellt ist. Der mobile Server 1 ist konfiguriert, um mit einem drahtlosen Zugangspunkt 2, auch Hot-Spot genannt, zu kommunizieren. Der drahtlose Zugangspunkt (WAP = wireless access point) ist ein Netzknoten für ein drahtloses lokales Netzwerk (wireless local area networt = WLAN). Über den drahtlosen Zugangspunkt 2 können Tablet PCs 3 Daten mit dem mobilen Server 1 und untereinander austauschen. Die Tablet PCs 3 sind hierfür mit einer handelsüblichen WLAN-Karte ausgestattet, das heißt einer drahtlosen Netzwerkszugangskarte, die gemäß einem gängigen Standard für drahtlose Datennetzwerke, z.B. IEEE 802.11g Wireless Standard, mit dem Zugangspunkt 2 kommunizieren. Darüber hinaus verfügt ein Tablet PC 3 über einen berührungsempfindlichen Bildschirm 4, einen sogenannten Touch-Screen. Der Touch-Screen ist meist ein hintergrundbeleuchteter LCD-Flachbildschirm mit berührungsempfindlicher Oberfläche. Es kommen aber auch alle anderen Bildschirmtechniken in Frage, die in einem leichten mobilen Gerät integrierbar sind.

An dem den Bildschirm 4 umgebenden Rahmen sind weitere Bedienelemente 5 des Tablet PC 3 angebracht.

Der Tablet PC 3 verfügt über die üblichen Baugruppen eines mobilen Personalcomputers (Prozessor = CPU, Arbeitsspeicher = RAM, Grafikkarte, Festplattenlaufwerk, aufladbare Batterie für die Stromversorgung, etc.). Dadurch, daß der Tablet PC 3 über ein Funknetz mit dem Server 1 kommuniziert, behindert er den Beobachter der den Tablet PC 3 nutzt nicht in seiner Bewegungsfreiheit. Jeder Beobachter verfügt über einen eigenen Tablet PC 3, der über die Kommunikation seiner WLAN-Karte mit dem drahtlosen Zugangspunkt (Hot-Spot) 2 kommuniziert und dadurch sowohl mit dem Server 1 als auch mit den anderen Tablet PCs 3 anderer Beobachter vernetzt ist.

Der mobile Server 1 selbst ist vorzugsweise über das Internet mit einer Datenbank 6 auf einem zentralen Server 7 verbunden. Der zentrale Server 7 kann beispielsweise einem Personalberatungsunternehmen oder der Personalabteilung eines Großunternehmens zugeordnet sein. Die Datenbank 6 des zentralen Servers 7 enthält alle für die verschiedenen Personaltests des betroffenen Unternehmen relevanten Daten, insbesondere vorbereitete Eingabemasken, welche auf den Tablet PCs während der Erfassung der Testergebnisse angezeigt werden und Eingabefelder enthalten, in denen der Beobachter die Ergebnisse eintragen kann. In der Datenbank des zentralen Servers 7 können auch Ergebnisse früherer Tests abgespeichert sein. Der zentrale Server 7 ist über ein eigenes lokales Datennetzwerk mit mehreren Arbeitsplatzrechnern (auch Workstations genannt) 8 verbunden. An den Arbeitsplatzrechnern 8 können Mitarbeiter des Unternehmens die Daten auf dem zentralen Server 7 abrufen, bearbeiten und abspeichern.

Schließlich zeigt die Figur 1 eine Videokamera 10, die mit einem Video-Aufzeichnungsträger 11 verbunden ist, auf den Videoaufnahmen des Tests abgespeichert werden. Vorzugsweise werden digitale Videoaufzeichnungen erstellt, welche mit einem Zeitsignal abgespeichert werden. Auf diese Weise ist es möglich, den Zeitpunkt im Test den über die Tablet PCs erfaßten Zeitpunkten bestimmter Eintragungen zuzuordnen. Als Aufzeichnungsträger 11 kann beispielsweise eine Magnetbandkassette oder vorzugsweise eine Festplatte mit großer Speicherkapazität verwendet werden.

Durch die Videoaufnahme ist es auch möglich, daß mindestens ein Beobachter zeitversetzt die getestete Person beurteilt, wobei auch hier automatisch die Testzeit erfaßt wird. Als Zeitpunkt des Testbeginns wird dann der Beginn der Wiedergabe der Videoaufnahme angenommen, so daß die Zeitpunkte der Eintragungen den Zeitpunkten der tatsächlich während des Tests durchgeführten Eintragungen anderer Beobachter zugeordnet werden können. Auch erlaubt das Zeitsignal der digitalen Videoaufnahme eine Wiedergabe der Einträge der Beobachter gemeinsam mit der Wiedergabe der Videoaufnahme, wobei die Zeitpunkte der Einträge den Zeiten der Videoaufnahme zugeordnet sind. Es können zum Beispiel am Rand eines Videobildes der Videoaufnahme Bildsegmente erzeugt werden, die bestimmten Beobachtern zugeordnet sind und deren Einträge in eine Eingabemaske während der Testdurchführung anzeigen. Es können hierdurch nicht nur die Eintragungen anhand der Videoaufzeichnungen überprüft werden, sondern auch die Leistungen der Beobachter überprüft und ggf. im Rahmen einer Schulung verbessert werden.

Die digitale Videoaufzeichnung wird bei dem System aus Fig. 1 über das Internet auf die Datenbank 6 des Servers 7 übertragen. Sie kann bei Bedarf auch über den mobilen Server 1 abgerufen werden. Bei breitbandigem Zugang in das Internet können die Daten eines digitalen Videosignals nahezu in Echtzeit übertragen werden. In der Figur 1 ist nur eine Videokamera 10 dargestellt. Es ist selbstverständlich, daß mehrere Videokameras 10 gleichzeitig betrieben werden können und so unterschiedliche Perspektiven des Tests oder unterschiedliche Testräume aufnehmen können.

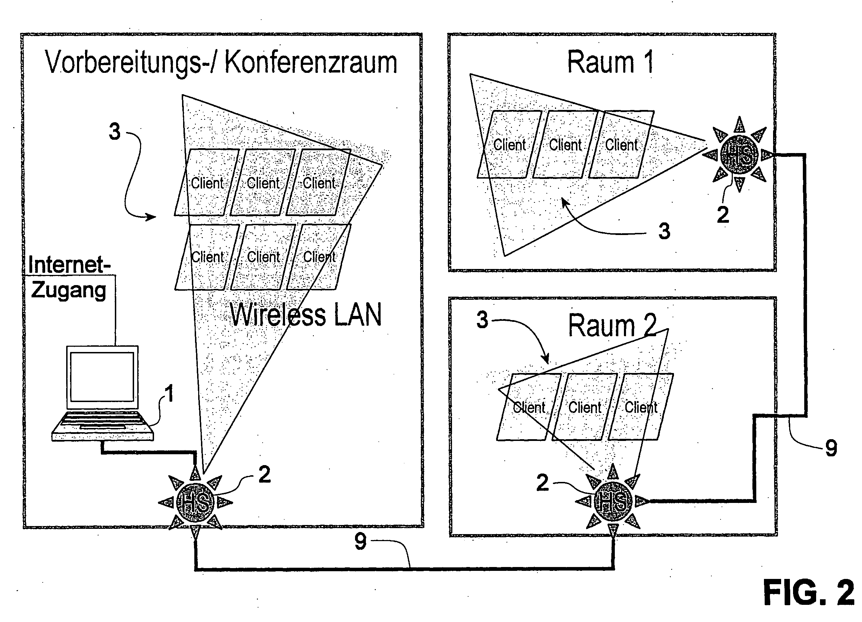

Die Figur 2 zeigt die Vernetzung der einzelnen Computer des erfindungsgemäßen Systems für den Betrieb eines Assessment-Centers. Der mobile Server 1 wird in einem Vorbereitungsraum oder Konferenzraum aufgestellt. Er ist vorzugsweise über einen Internet-Zugang an das Internet angeschlossen, so daß er auf Daten des zentralen Servers 7 (siehe Figur 1) zugreifen kann. An dem mobilen Server 1 ist ein Hot-Spot 2, auch drahtloser Zugangspunkt (wireless access point) genannt, angeschlossen. Mit dem Hot-Spot 2 im Konferenzraum sind über Datenleitungen 9 zwei weitere Hot-Spots 2 in unterschiedlichen Räumen verbunden. Jedes für die Datenübertragung geeignete Kabel kann als Datenleitung 9 verwendet werden. Übliche Netzwerkkabel werden heute als Koaxial-Kabel oder Twisted-Pair-Kabel ausgeführt. Es ist aber auch möglich, eine Datenverbindung über Stromleitungen des Stromnetzes eines Gebäudes herzustellen. Schließlich können auch Funkverbindungen zwischen den verschiedenen Hot-Spots 2 des Netzwerks eingerichtet werden.

Es ist darauf zu achten, daß alle Bereiche der Räume eines Assessment-Centers, in denen sich Beobachter möglicherweise aufhalten, durch mindestens einen der Hot-Spots 2 zur Durchführung der Datenübertragung abgedeckt sind. Die verschiedenen Hot-Spots 2 kommunizieren mit den WLAN-Karten in den Tablet PCs 3, auf denen ein Client-Programm des auf dem Server 1 ablaufenden Programms läuft. Das auf dem Server 1 ablaufende Programm wird im Rahmen dieser Anmeldung als "Digital Assessment Center" bezeichnet.

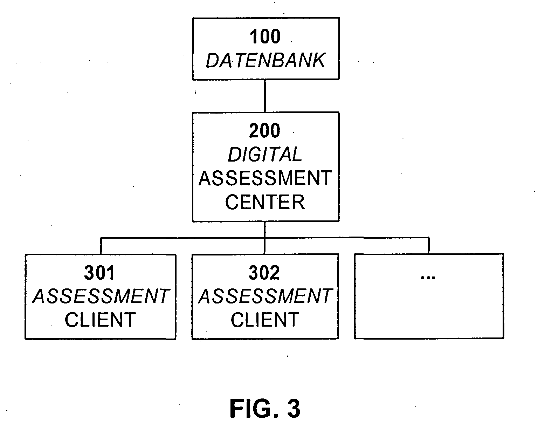

Die Figur 3 zeigt die verschiedenen Programm-Module, die zur Durchführung der erfindungsgemäßen Personalbeurteilung verwendet werden.

Es besteht zum einen aus einer Datenbank 100, die auf dem mobilen Server 1 (siehe Figuren 1 und 2) abgespeichert ist. Die Datenbank 100 kann die gleichen Informationen wie die Datenbank 6 (Figur 1) auf dem zentralen Server 7 des Unternehmens enthalten. Sie kann sich aber auch von dieser zentralen Datenbank 6 unterscheiden, da zur Durchführung des erfindungsgemäßen Verfahrens eine Anbindung an die zentrale Datenbank zwar nützlich aber nicht unbedingt erforderlich ist. In diesem Fall erfolgt vorzugsweise von Zeit zu Zeit ein Abgleich der Daten in der Datenbank 100 des mobilen Servers 1 mit den Daten in der Datenbank 6 des zentralen Servers 7. Auf dem mobilen Server 1 (Figuren 1 und 2) läuft das Datenerfassungsprogramm 200, welches als "Digital Assessment Center" bezeichnet wird. Auf jedem Tablet PC 3 eines Beobachters läuft ein Assessment-Client 301,302 ..., das heißt ein Programm, welches die Ergebniserfassung auf dem Tablet PC 3 (Figuren 1 und 2) steuert und die erfaßten Daten an das "Digital Assessment Center" 200 auf den mobilen Server 1 weiterleitet.

Das "Digital Assessment Center" auf dem mobilen Server 1 übernimmt die zentrale Steuerung der Ergebniserfassungsprogramme 301,302.... Es legt insbesondere den Testbeginn und das Testende fest und speichert, wie erwähnt, die verschiedenen Daten, welche von den Assessment-Clients 301, 302 ... übermittelt werden, ab. Die Zeiterfassung kann sowohl zentral im Digital Assessment Center 200 als auch dezentral in den einzelnen Assessment-Clients 301, 302 ... erfolgen. Eine dezentrale Zeiterfassung hat den Vorteil, daß der Assessment-Client 301, 302 ... auch dann funktionsfähig ist, wenn eine kurzzeitige Unterbrechung der drahtlosen Netzwerkverbindung mit dem mobilen Server 1 auftritt.

Jeder Assessment-Client 301, 302 erzeugt auf der Anzeigevorrichtung, nämlich dem berührungsempfindlichen Bildschirm jedes Tablet PCs 3 die Masken mit den für die Ergebniserfassung erforderlichen Eingabefeldern.

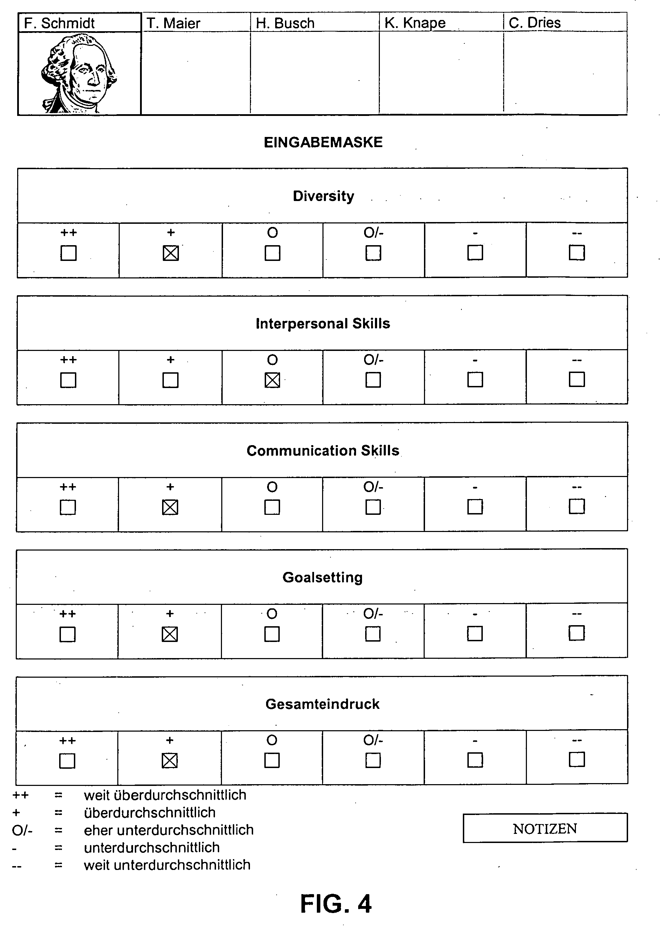

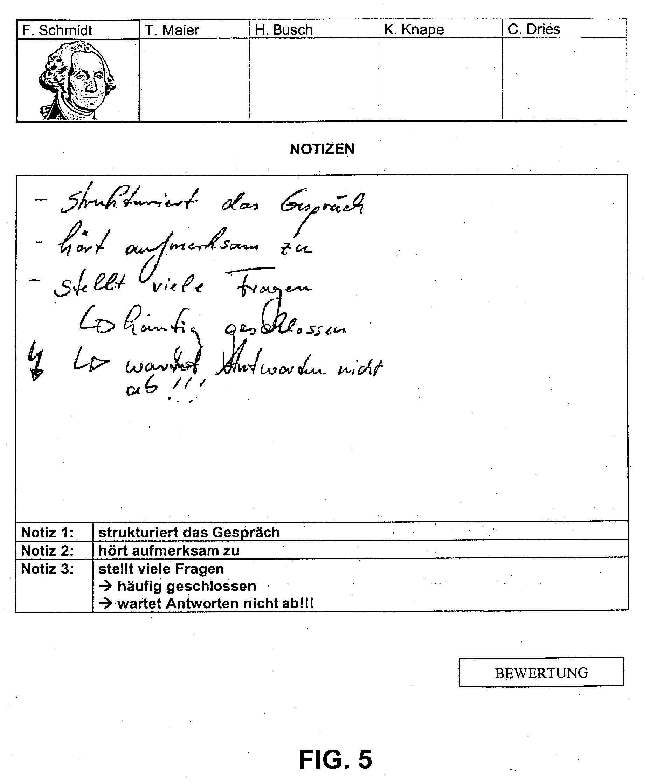

Die Figuren 4 und 5 zeigen Beispiele für derartige Eingabemasken. Dabei können die Eingabemasken durch sogenannte Fahnen am oberen Bildrand abwechselnd aktiviert werden, wenn zwischen ihnen hin und her geschaltet werden soll. Jede Eingabemaske ist einer bestimmten Person, die an dem Test teilnimmt, zugeordnet. Der Name der jeweiligen Person ist auf der Fahne der Eingabemaske eingetragen. Zusätzlich kann die Eingabemaske ein Feld aufweisen, in dem ein Bild der Person dargestellt ist. Auf diese Weise wird vermieden, daß Ergebnisse der Beobachter der falschen Person zugeordnet werden. In den Figuren 4 und 5 ist die Fahne der Person F. Schmidt in der linken Spalte aktiviert, so daß gleichzeitig das Bild des Herrn Schmidt gezeigt wird. Die Eingabemaske in Figur 4 ermöglicht die Eingabe von fünf verschiedenen Bewertungsstufen, welche von "weit überdurchschnittlich" bis "weit unterdurchschnittlich" reichen, und fünf verschiedenen Eigenschaften eines Bewerbers zugeordnet sind, welche durch den Beobachter bewertet werden sollen. Die den verschiedenen Eigenschaften zugeordneten Bewertungsstufen lassen sich durch sogenannte Kontrollkästchen aktivieren, welche unterhalb der den Bewertungsstufen zugeordneten Symbolen angeordnet sind. Durch ein derartiges Vorgehen lassen sich vergleichbare Beurteilungen durch verschiedene Beobachter erzielen.

Gemäß der Erfindung hält der Tablet PC 3, welcher als Computer für die Ergebniserfassung verwendet wird, nicht nur die zu jeder Eigenschaft gewählte Bewertungsstufe fest, sondern auch den Zeitpunkt, in dem die Bewertungsstufe durch den Beobachter ausgewählt wurde. Darüber hinaus kann der auf dem Tablet PC 3 laufende Assessment-Client 301, 302 ... auch Änderungen der Bewertungsstufen zu bestimmten Eigenschaften festhalten und die den Änderungen zugeordneten Zeitpunkte abspeichem. Hierdurch lassen sich Rückschlüsse auf Änderungen der Eindrücke der Beobachter im Laufe eines bestimmten Testes machen.

Auch kann das Programm so ausgelegt werden, daß zu einer bestimmten Eigenschaft mehrere Ergebnisse zugelassen werden. In diesem Fall können die in verschiedenen Zeitpunkten bestimmte Bewertungsstufen durch den Beobachter zu dieser Eigenschaft eingetragen werden. In der Praxis wird der Mittelwert aller Eingaben zu einer Eigenschaft gebildet. Hierdurch ist gewährleistet, daß die Eindrücke einer Person auf den Beobachter während der gesamten Testdauer erfaßt und berücksichtigt werden. Zusammen mit den eingegebenen Bewertungsstufen können durch den Computer die Zeitpunkte der jeweiligen Eingabe erfaßt werden.

Häufig sind die Testergebnisse in Form von Bewertungsstufen zu bestimmten Eigenschaften zu wenig aussagekräftig über einen bestimmten Bewerber. Aus diesem Grund bietet der auf dem Tablet PC 3 ablaufende Assessment-Client 301, 302 ... durch die in der in Figur 5 dargestellten alternative Eingabemaske die Möglichkeit der handschriftlichen Eingabe von Notizen. Diese Eingabemaske wird von einem Beobachter aktiviert, indem er auf der Eingabemaske in Figur 4 das unten links dargestellte Funktionsfeld mit dem Namen "NOTIZEN" mit einem Eingabestift berührt. Genauso kann der Beobachter durch Berührung des Funktionsfelds mit dem Namen "BEWERTUNG" in der Eingabemaske der Figur 5 zurück in die Eingabemaske der Figur 4 wechseln. Zwischen den verschiedenen beobachteten Personen wird durch Berühren der Namensfähnchen am oberen Rand der Eingabemaske umgeschaltet. Der Computer kann automatisch erfassen, wann ein Beobachter die Eingabemaske jeder der beobachteten Personen aktiviert hat. Hieraus können Rückschlüsse auf die Gesamtzeit gezogen werden, die sich ein Beobachter jeder der betrachteten Personen zugewandt hat.

Die Notizen werden über den berührungsempfindlichen Bildschirm 4 des Tablet PCs handschriftlich eingetragen. Ein Schrifterkennungsprogramm wandelt die handschriftlichen Einträge in alphanumerische Zeichen, vorzugsweise ASCII-Codes, um. Das Ergebnis der Schrifterkennung ist am unteren Rand der in Figur 5 dargestellten Eingabemaske zu erkennen. Da die Schrifterkennung in vielen Fällen noch nicht fehlerfrei arbeitet, können neben den ASCII-Codes der Schrifterkennung auch digitale Bilddaten der handschriftlichen Notizen abgespeichert werden. Dies ermöglicht eine spätere Kontrolle der durch die Schrifterkennung erkannten Notizen.

Die in Figur 5 dargestellte Eingabemaske kann auch zur handschriftlichen Eingabe einer Nachricht verwendet werden. In diesem Fall ist sie mit weiteren auf dem Bildschirm 4 darzustellenden Steuerungsfeldern zu koppeln, welche das Versenden der Nachricht an einen bestimmten Computer oder an eine Gruppe von Computern des Netzwerkes veranlassen. Jeder Computer, der mit dem Netzwerk des Assessment Centers verbunden ist, erhält eine eindeutige Adresse und Textmeldungen können beispielsweise nach Art des E-Mail-Versands von einem Computer des Netzwerks zum anderen versandt werden. Bei Empfang der Nachricht erzeugt der empfangende Computer auf seinem Bildschirm ein Anzeigefenster, auf dem die Nachricht für den Beobachter gut lesbar angezeigt wird.

Auch wenn der Gegenstand der Erfindung in Verbindung mit mehreren konkreten Ausführungsformen beschrieben wurde, ist die Erfindung nicht auf diese konkreten Ausführungsformen beschränkt, sondern umfaßt vielmehr alle Alternativen, Abwandlungen und Äquivalente innerhalb des durch die Ansprüche definierten Schutzumfangs.

### Bezugszeichenliste:

- 1: Mobiler Server
- 2: drahtloser Zugangspunkt
- 3: Tablet PC, Computer
- 4: berührungsempfindlicher Bildschirm, Anzeigevorrichtung
- 5: Bedienungselemente
- 6: Datenbank
- 7: Server
- 8: Arbeitsplatzrechner
- 9: Datenleitung
- 10: Videokamera
- 11: Aufzeichnungsträger

## Patentansprüche

1. Verfahren zur Erfassung der Ergebnisse eines psychologischen Tests, insbesondere eines Tests zur Personalbeurteilung, durch mindestens einen Beobachter, **dadurch gekennzeichnet, daß** auf einer Anzeigevorrichtung eines Computers Eingabefelder erzeugt werden, der Beobachter die Ergebnisse in die Eingabefelder einträgt, die Ergebnisse in einem Datenspeicher abgespeichert werden und der Computer mindestens eine zusätzliche Funktion der Ergebniserfassung und/oder Testdurchführung automatisch durchführt.

2. Verfahren nach Anspruch 1, **dadurch gekennzeichnet, daß** der Computer die seit Testbeginn abgelaufene Zeit des Tests erfaßt.

3. Verfahren nach Anspruch 1 oder 2, **dadurch gekennzeichnet, daß** der Computer zusammen mit einem Eintrag in ein Eingabefeld die im Zeitpunkt des Eintrags abgelaufene Zeit des Tests abspeichert.

4. Verfahren nach einem der vorangehenden Ansprüche, **dadurch gekennzeichnet, daß** der Wert des Eintrag mit dem Zeitpunkt des Eintrags gewichtet wird.

5. Verfahren nach einem der vorangehenden Ansprüche, **dadurch gekennzeichnet, daß** der Computer ein Eingabefeld erst nach einer vorbestimmten Zeit für die Aufnahme eines Eintrags freigibt.

6. Verfahren nach einem der vorangehenden Ansprüche, **dadurch gekennzeichnet, daß** das Ergebnis zu einem bestimmten Kriterium in Form eines skalierten Wertes erfaßt wird und daß zu diesem Kriterium mehrere zu unterschiedlichen Zeiten eingegebene Ergebnisse erfaßt werden.

7. Verfahren nach Anspruch 6, **dadurch gekennzeichnet, daß** die Summe aller zu dem genannten Kriterium eingegebenen Ergebnisse gebildet und durch die Anzahl der Eingaben geteilt wird.

8. Verfahren nach Anspruch 6 oder 7, **dadurch gekennzeichnet, daß** alle zu dem genannten Kriterium eingegebenen Ergebnisse zusammen mit dem Zeitpunkt ihrer Eingabe abgespeichert werden.

9. Verfahren nach einem der vorangehenden Ansprüche, **dadurch gekennzeichnet, daß** mehrere Beobachter zur Erfassung der Testergebnisse vorhanden sind und daß jeder Beobachter seine Ergebnisse auf einem eigenen Computer einträgt, wobei die Computer der Beobachter untereinander vemetzt sind.

10. Verfahren nach Anspruch 9, **dadurch gekennzeichnet, daß** die Testergebnisse der Computer aller Beobachter in eine gemeinsame Datenbank auf einem Server geschrieben werden.

11. Verfahren nach Anspruch 10, **dadurch gekennzeichnet, daß** aus der gemeinsamen Datenbank automatisch eine Ergebnisdatei erstellt wird.

12. Verfahren nach einem der Ansprüche 9 bis 11, **dadurch gekennzeichnet, daß** mindestens ein Computer eine Nachricht über ein Datennetzwerk versendet, die von mindestens einem anderen Computer empfangen und wiedergegeben wird.

13. Verfahren nach Anspruch 12, **dadurch gekennzeichnet, daß** die zu versendende Nachricht in ein Absendefeld auf dem Bildschirm des versendenden Computers eingegeben wird.

14. Verfahren nach Anspruch 12 oder 13, **dadurch gekennzeichnet, daß** die empfangene Nachricht in einem Anzeigefeld auf dem Bildschirm des empfangenden Computers wiedergegeben wird.

15. Verfahren nach einem der vorangehenden Ansprüche, **dadurch gekennzeichnet, daß** die Eingaben über einen berührungsempfindlichen Bildschirm des Computers eingegeben werden.

16. Verfahren nach Anspruch 15, **dadurch gekennzeichnet, daß** die Eingaben handschriftlich erfolgen.

17. Verfahren nach Anspruch 16, **dadurch gekennzeichnet, daß** die handschriftlichen Eingaben als Bilddatensatz abgespeichert wird.

18. Verfahren nach Anspruch 16 oder 17, **dadurch gekennzeichnet, daß** die handschriftlichen Eingaben durch eine automatische Schrifterkennung in alphanumerischen Text umgewandelt und als Textdatensatz abgespeichert wird.

19. Verfahren nach einem der vorangehenden Ansprüche, **dadurch gekennzeichnet, daß** ein Beobachter während eines Tests mehrere Personen beobachtet, wobei der Computer dieses Beobachters für jede der genannten Personen eine separate Eingabemaske mit Eingabefeldern erzeugt, wobei der Beobachter jede der Masken durch eine Eingabe in den Computer aktivieren kann.

20. Verfahren nach Anspruch 19, **dadurch gekennzeichnet, daß** jede der Eingabemasken den Namen einer bestimmten Person, der sie zugeordnet ist, in einem Identifizierungsfeld anzeigt.

21. Verfahren nach Anspruch 19 oder 20; **dadurch gekennzeichnet, daß** jede der Eingabemasken ein Bild einer bestimmten Person, der sie zugeordnet ist, in einem Identifizierungsfeld anzeigt.

22. Verfahren nach einem der Ansprüche 19 bis 21, **dadurch gekennzeichnet, daß** der Computer für jede Eingabemaske die Gesamtzeit erfaßt, in der diese Eingabemaske durch den Beobachter während eines Tests aktiviert ist.

23. Verfahren nach Anspruch 22, **dadurch gekennzeichnet, daß** die Eingaben eines Beobachters zu einer bestimmten Person mit dem Betrag der Gesamtzeit, in der die der genannten Person zugeordnete Eingabemaske durch den Beobachter aktiviert war, gewichtet wird.

24. Verfahren nach einem der vorangehenden Ansprüche, **dadurch gekennzeichnet, daß** mindestens eine Videoaufnahme von der Durchführung des Tests aufgezeichnet wird.

25. Verfahren nach Anspruch 24, **dadurch gekennzeichnet, daß** der Beobachter die Videoaufnahme betrachtet und dabei die Ergebnisse in die Eingabefelder einträgt.

26. Verfahren nach Anspruch 25, **dadurch gekennzeichnet, daß** die Videoaufnahme zeitversetzt abgespielt wird und wobei der Testbeginn mit dem Beginn des Abspielens der Videoaufnahme gleichgesetzt wird.

27. Verfahren nach einem der Ansprüche 24 bis 16, **dadurch gekennzeichnet, daß** die Videoaufnahme zusammen mit einer Darstellung der Eintragungen mindestens eines Beboachters in seinen Computer auf einer Anzeigevorrichtung wiedergegeben wird.

28. Computerprogrammprodukt, welches zur Erfassung der Ergebnisse eines psychologischen Tests direkt in den Arbeitsspeicher eines Computers ladbar ist und Programmcodeabschnitte aufweist, welche beim Ablauf des Programms die Schritte des Verfahrens nach einem der vorangehenden Ansprüche ausführen.

29. Computersystem zur Erfassung der Ergebnisse eines psychologischen Tests, insbesondere eines Tests zur Personalbeurteilung, durch mindestens einen Beobachter, **dadurch gekennzeichnet, daß** es für mehrere Beobachter jeweils einen Computer (3) umfaßt, auf dem ein Computerprogrammprodukt nach Anspruch 28 abläuft.

30. Computersystem nach Anspruch 29, **dadurch gekennzeichnet, daß** der Computer (3) einen Timer umfaßt.

31. Computersystem nach Anspruch 29 oder 30, **dadurch gekennzeichnet, daß** der Computer (3) einen berührungsempfindlichen Bildschirm (4) aufweist.

32. Computersystem nach einem der Ansprüche 29 bis 31, **dadurch gekennzeichnet, daß** der Computer (3) ein Modul zur drahtlosen Datenübertragung aufweist.

33. Computersystem nach einem der Ansprüche 29 bis 32, **dadurch gekennzeichnet, daß** es einen lokalen Server (1) umfaßt, der mit einem drahtlosen Zugangspunkt (2) verbunden ist.

34. Computersystem nach Anspruch 33, **dadurch gekennzeichnet, daß** der lokale Server (1) mit einem zentralen Server (7) über ein Datennetzwerk, insbesondere das Internet, verbunden ist.

35. Computersystem nach einem der Ansprüche 29 bis 34, **dadurch gekennzeichnet, daß** es mit einer den Test aufnehmenden Videokamera (10) gekoppelt ist, so daß die durch das Computersystem erfaßte abgelaufene Zeit des Tests mit der Aufnahmezeit der Videokamera (10) synchronisiert ist.

36. Verwendung eines Computers (3) zur Erfassung der Ergebnisse eines psychologischenTests, insbesondere eines Tests zur Personalbeurteilung, durch mindestens einen Beobachter und zur automatischen Durchführung mindestens einer zusätzlichen Funktionen der Ergebniserfassung und/oder Testdurchführung.
